(19) Europäisches Patentamt
European Patent Office
Office européen des brevets

(11) Numéro de publication: **0 156 710**
**B1**

# (12) FASCICULE DE BREVET EUROPEEN

(45) Date de publication du fascicule du brevet: **10.12.86**

(51) Int. Cl.⁴: **C 07 D 251/34**, C 08 F 20/36

(21) Numéro de dépôt: **85400432.2**

(22) Date de dépôt: **06.03.85**

(54) **Nouveau dérivé diallylique de l'acide cyanurique.**

(30) Priorité: **09.03.84 FR 8403606**

(43) Date de publication de la demande: **02.10.85 Bulletin 85/40**

(45) Mention de la délivrance du brevet: **10.12.86 Bulletin 86/50**

(84) *Etats contractants désignés:* **AT BE CH DE GB IT LI LU NL SE**

(56) Documents cités:
**EP - A - 0 052 800**
**US - A - 2 934 525**
**US - A - 3 332 946**

(73) Titulaire: **Société Chimique des Charbonnages S.A., Tour Aurore Place des Reflets, F-92080 Paris La Défense Cédex 5 (FR)**

(72) Inventeur: **Teissier, Rémy, 11 Allée de Brienne, F-31000 Toulouse (FR)**
Inventeur: **Clamens, Serge, 1 rue Jean d'Alembert, F-31100 Toulouse (FR)**

(74) Mandataire: **Rieux, Michel et al, C d F Chimie S.A. Service Propriété Industrielle Tour Aurore Place des Reflets Cédex no. 5, F-92080 Paris la Défense 2 (FR)**

EP 0 156 710 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne le diallyl monoacryloyl oxyéthyl isocyanurate et des procédés de fabrication.

On connaît de nombreux dérivés organiques de l'acide cyanurique. Ce sont des dérivés triaziniques substitués sur l'azote de formule

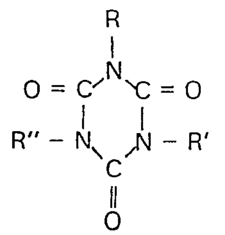

Le triallyle isocyanurate dans lequel les trois radicaux R, R' et R'' sont des radicaux $CH_2 = CH - CH_2$ est fabriqué et commercialisé.

On le prépare par action de chlorure d'allyle sur le cyanurate trisodique. Il est utilisé comme réticulant en particulier dans les polyesters.

Le diallyle isocyanurate est lui aussi connu. Il se prépare en particulier par action du chlorure d'allyle sur un sel disodique de l'acide cyanurique en milieu aqueux, en présence de chlorure cuivreux comme catalyseur (voir brevet de l'Allemagne de l'Est N° 51 858).

Parmi les dérivés trisubstitués connus de l'acide cyanurique, on peut ajouter les dérivés isocyanuriques méthacryliques ou méthacryliques-maléiques, c'est-à-dire les dérivés de l'acide cyanurique dont les substituants R, R' et R'' sont identiques et ont pour formule $-CH_2 - C(R_o)(R)H$ avec $R_o$ qui est un atome d'hydrogène, un radical méthyle ou éthyle et R qui est:
$-OH, - O - C(O) - C(CH_3) = CH_2$ ou
$-O-C(O)-CH = CH-C(O)-O-CH_2-CH(OH)$
$-CH_2-O-C(O)-C(CH_3) = CH_2$. Ces dérivés sont utilisés dans les adhésifs (voir brevet européen n° 52 800).

Le sel sodique ou potassique du diallyle isocyanurate se prépare facilement en milieu aqueux par dissolution à froid de diallyle isocyanurate dans une solution de base alcaline en quantité stœchiométrique et ensuite par évaporation de l'eau. Ces sels alcalins sont des produits intermédiaires dans de nombreuses synthèses.

On a déjà préparé des dérivés monoalcoylés diallyliques de l'acide cyanurique. Ils ont la formule suivante:

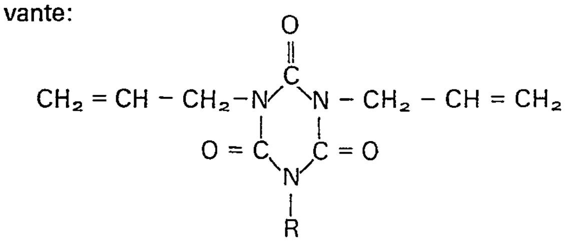

Les composés préparés sont ceux où R est un radical éthyle, carboéthoxyméthyle, (β-cyano) éthyle, β-hydroxyéthyle, (β-bromo) éthyle et β-dioxypropyle. De tels composés sont par exemple décrits dans un article de K.K. KHONERVA et al. dans ZIN. FIZ. KHIM. POLIM. 1971 n° 9, pages 30 à 33, dans les brevets américains 3,200,119 et 3,332,946 et dans le brevet anglais 961 624. Il est connu que ces produits peuvent être polymérisés.

Parmi les dérivés diallyliques de l'acide cyanurique, on peut ajouter les produits dans lesquels R est un hydroxyalkyle. Ce sont des réticulants allyliques solubles dans l'eau qui sont utilisés notamment pour la préparation de revêtements, d'adhésifs ou d'élastomères (voir brevet US 3,332,946).

La présente invention concerne un nouveau composé de formule:

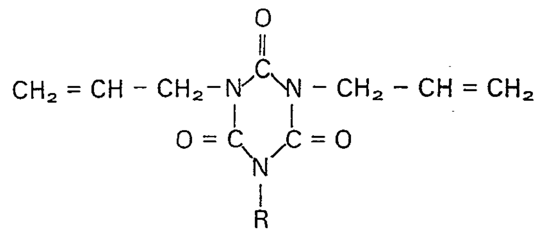

dans lequel R est un radical acryloyl oxyéthyle.

Ce nouveau composé a pour structure:

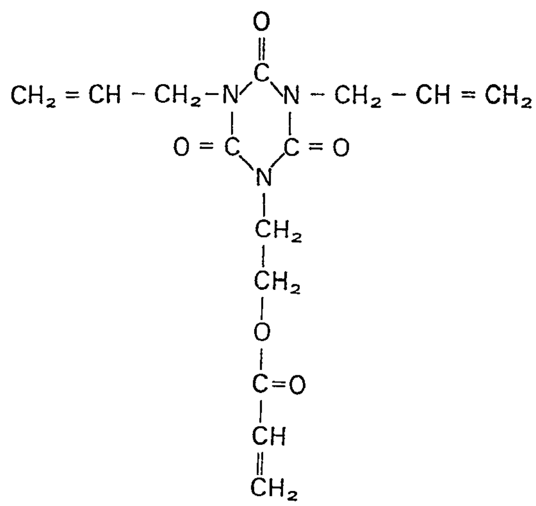

Les spectres infrarouges et RMN (voir figure annexée) confirment cette structure. Ce composé peut s'appeler: diallyl monoacryloyl oxyéthyl isocyanurate ou 1,3 di (2 propényl) 5 acryloyl oxyéthyl 1, 3, 5 triazine - 2, 4, 6 (1H, 3H, 5H) trione.

Il a pour formule brute $C_{14}H_{17}N_3O_5$. Son poids moléculaire est de 307,3. La température d'ébullition est de 170° C sous 0,5 mm de Hg. L'indice de réfraction $n_0^{20}$ est de 1,5145, mesuré au réfractomètre d'Albe. A température ambiante, il se présente comme un liquide visqueux transparent.

Le diallyl monoacryloyl oxyéthyl isocyanurate peut se polymériser. Il peut également se copolymériser avec des monomères acryliques. La copolymérisation s'effectue «en masse» en présence d'initiateurs peroxydiques avec différentes proportions de monomères. Il peut être utilisé comme agent de réticulation dans les polymères tels que les polyéthylènes, polystyrènes, polymères polyalkyliques et polyacryliques. Il confère à ces derniers des propriétés ignifuges.

Le diallyl monoacryloyl oxyéthyl isocyanurate est préparé de différentes façons. Selon un premier mode de fabrication, on prépare le sel de sodium

du diallyl isocyanurate; on fait réagir sur ce sel la chlorhydrine ou la bromhydrine de glycol ou l'oxyde d'éthylène dans un solvant, en particulier le diméthylformamide. La réaction s'écrit:

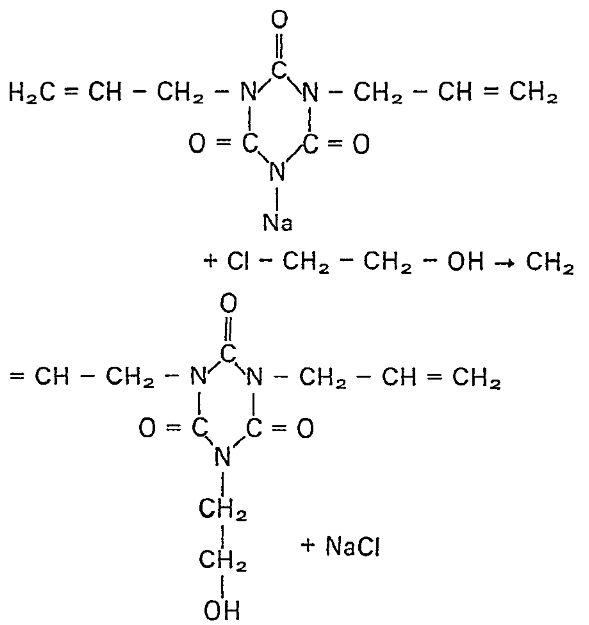

Le diallyl mono hydroxyéthyl isocyanurate ainsi obtenu est estérifié par l'acide acrylique pour donner le diallyl monoacryloyl oxyéthyl isocyanurate qui est séparé du mélange réactionnel par distillation sous vide.

Selon un second procédé de fabrication, on fait réagir l'acrylate de β-chloroéthyle (qui est commercialisé) avec le sel de sodium de l'isocyanurate de diallyle en présence de diméthylformamide ou d'eau. On sépare le diallyl monoacryloyl oxyéthyl isocyanurate formé par distillation sous vide par décantation ou par extraction à l'aide d'un solvant tel que toluène ou éther.

Les exemples donnés ci-dessous à titre indicatif permettront de mieux comprendre l'invention.

*Exemple 1:*

a) *Préparation de monohydroxyéthyle diallyle isocyanurate*

Dans un réacteur agité muni d'un condenseur à reflux on introduit la charge suivante:

– 231 g soit 1 mole de diallyle isocyanurate de sodium,

– 82 g soit 1,018 mole de chlorhydrine de glycol,

– 800 ml de diméthylformamide comme solvant.

On porte sous agitation le mélange réactionnel au reflux. Celui-ci commence à 140° C. Au bout de trois heures, la température de reflux est de 158° C. On arrête le chauffage et on refroidit le mélange réactionnel. On filtre le précipité composé de NaCl et de traces de diallyle isocyanurate n'ayant pas réagi. Le taux de réaction est très voisin de 100%. Le filtrat est stabilisé par 100 ppm d'hydroquinone et il est ensuite distillé sous vide. On distille d'abord le diméthylformamide qui passe à 23° C sous 0,9 mm de Hg, puis on récupère le diallyl monohydroxy éthyl isocyanurate qui distille entre 160 et 173° C sous 0,9 mm de Hg.

Le diallyl monohydroxyéthyl isocyanurate se présente sous forme d'un produit visqueux qui peu à peu durcit et devient cireux. Le produit cireux a les caractéristiques suivantes:

– point de fusion: 35° C,

– dosage des OH 6,36% (théorie 6,7%),

– indice de brome: 126,0 (théorie 126,4) (dosage des doubles liaisons).

L'analyse élémentaire donne les résultats suivants:

– C: 52,11% (théorie 52,17%),

– H: 6,01% (théorie 5,93%),

– N: 16,68% (théorie 16,60%).

b) *Estérification de l'alcool:*

Dans un réacteur muni d'une agitation, d'une prise de température, d'un extracteur d'eau par azéotropie, décantation et recyclage du solvant, on introduit la charge suivante:

– 253 g (1 mole) de diallyl mono hydroxyéthyl isocyanurate,

– 74 g (1,02 mole) d'acide acrylique,

– 100 ml de toluène comme solvant,

– 1 g d'acide sulfurique ou d'acide paratoluène sulfonique comme catalyseur,

– 1 g d'éther méthylique de l'hydroquinone comme stabilisant pour empêcher la polymérisation.

On porte le mélange réactionnel au reflux sous pression réduite (400 mm de Hg).

On extrait l'eau qui se forme par distillation azéotropique, décantation et recyclage du toluène décanté vers la réaction. Au bout de 2 h 30, on a extrait 16 g d'eau.

On distille le toluène puis on distille sous vide le diallyl monoacryloyloxyéthyl isocyanurate qui passe entre 165 et 170° C sous 0,5 mm de Hg.

On recueille 200 g de diallyle monoacryloyloxyéthyl isocyanurate. Le rendement est de 65%.

*Exemple 2* (Transestérification)*:*

Dans un réacteur agité muni d'une colonne à distiller, on introduit:

– 258 g (3 moles) d'acrylate de méthyle,

– 253 g (1 mole) de diallyl monohydroxyéthyl isocyanurate,

– 1 g de titanate d'éthyle comme catalyseur,

– 1 g d'éther méthylique de l'hydroquinone comme stabilisant.

On chauffe au reflux sous pression réduite (400 mm de Hg) et distille à 60° C; peu à peu, l'azéotrope méthanol/acrylate de méthyle 70/30.

Au bout de 2 à 2 h 30, on a recueilli environ 30 g de méthanol. On distille alors l'acrylate de méthyle excédentaire, puis sous vide l'alcool et enfin le diallyl monoacryloyl oxyéthyl isocyanurate. Le rendement est de 65%.

*Exemple 3* (Réaction du diallyle isocyanurate de Na)*:*

Dans un réacteur muni d'un condenseur à reflux, d'une prise de température, d'un dispositif de bullage d'air et d'une agitation, on introduit:

– 239 g de diallyle isocyanurate de Na (1 mole),
– 96 g d'acrylate de β-chloroéthyle (1,2 mole),
– 1400 ml de diméthylformamide,
– 6 g d'éther méthylique d'hydroquinone.

On chauffe à reflux pendant 2 heures. La température finale du reflux est de 150° C. On refroidit le mélange réactionnel et on filtre le NaCl formé.

On distille le diméthylformamide puis on recueille sous un vide de 0,5 mm de Hg le diallyl monoacryloyl oxyéthyl isocyanurate qui passe entre 160 et 170° C.

On obtient 265 g de diallyl monoacryloyl oxyéthyl isocyanurate. Le rendement est de 85%.

*Exemple 4:*

On opère comme dans l'exemple 3 sauf que le diméthylformamide est remplacé par de l'eau. On chauffe sous reflux pendant 4 à 5 heures puis on refroidit. On sépare par décantation 300 g de diallyl monoacryloyl oxyéthyl isocyanurate que l'on purifie par distillation sous vide.

*Exemple 5* (Application)*:*

On mélange 100 parties de diallyl monoacryloyl oxyéthyl isocyanurate avec 3 parties de benzophénone. On applique le mélange obtenu sur une plaque d'ABS. Après irradiation par une lampe UV pendant 2 heures, on obtient un revêtement photosensible qui possède une dureté remarquable, une bonne adhérence à l'ABS. L'adjonction de 2 parties de $(CH_3)_2N-CH_2-CH_2-OH$ raccourcit le temps de cuisson.

## Revendications

1. Le diallyl monoacryloyl oxyéthyl isocyanurate de formule

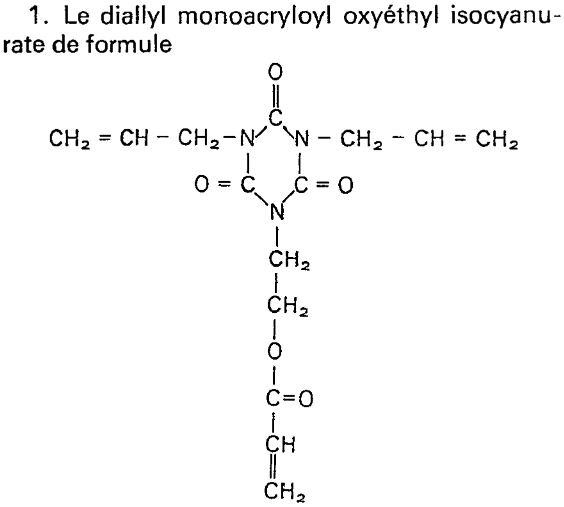

2. Procédé de préparation du composé selon la revendication 1, caractérisé par le fait que l'on fait réagir le sel sodique ou potassique du diallyl isocyanurate avec la chlorhydrine ou la bromhydrine de glycol ou l'oxyde d'éthylène dans un solvant, le diallyl monohydroxyéthyl ainsi obtenu est estérifié par l'acide acrylique et on sépare le diallyl monoacryloyl oxyéthyl isocyanurate par distillation sous vide.

3. Procédé de préparation du composé selon la revendication 1, caractérisé par le fait que l'on fait réagir l'acrylate de β-chloroéthyle avec le sel de sodium de l'isocyanurate du diallyle en présence de diméthylformamide ou d'eau et on sépare le diallyl monoacryloyl oxyéthyl isocyanurate ainsi formé.

## Patentansprüche

1. Diallylmonoacryloyloxyäthylisocyanurat der Formel

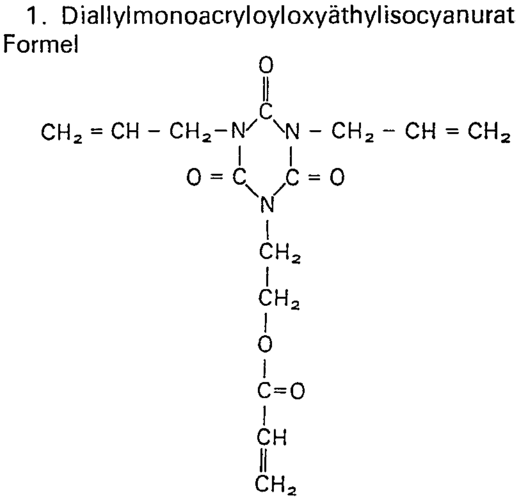

2. Verfahren zur Herstellung der Verbindung nach Anspruch 1, dadurch gekennzeichnet, dass man das Natrium – oder Kalisalz von Diallylisocyanurat mit Glykolchlorhydrin oder Glykolbromhydrin oder Äthylenoxid in einem Lösungsmittel zur Reaktion bringt, das so erhaltene Diallylmonohydroxyäthyl mit Acrylsäure verestert und das Diallylmonoacryloyloxyäthylisocyanurat durch Vakuumdestillation abtrennt.

3. Verfahren zur Herstellung der Verbindung nach Anspruch 1, dadurch gekennzeichnet, dass man β-Chloräthylacrylat mit dem Natriumsalz von Diallylisocyanurat in Gegenwart von Dimethylformamid oder Wasser zur Reaktion bringt und das so gebildete Diallylmonoacryloyloxyäthylisocyanurat abtrennt.

## Claims

1. Diallyl monoacrylol oxyethyl isocyanurate with the formula:

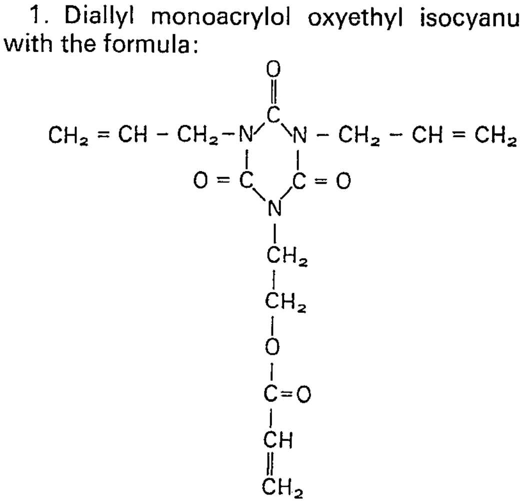

2. A method for preparing the compound according to claim 1, characterised in that the sodium or potassium salt of the diallyl isocyanurate is

reacted with the glycol hydrochloride or hydrobromide or ethylene oxide in a solvent, the diallyl monohydroxyethyl thus obtained is esterified using the acrylic acid and the diallyl monoacrylol oxyethyl isocyanurate is separated by distillation in a vacuum.

3. A method for preparing the compound according to claim 1, characterised in that the β-chloroethyl acrylate is reacted with the diallyl isocyanurate sodium salt in the presence of dimethylformamide or water and the diallyl monoacrylol oxyethyl isocyanurate thus formed is separated.

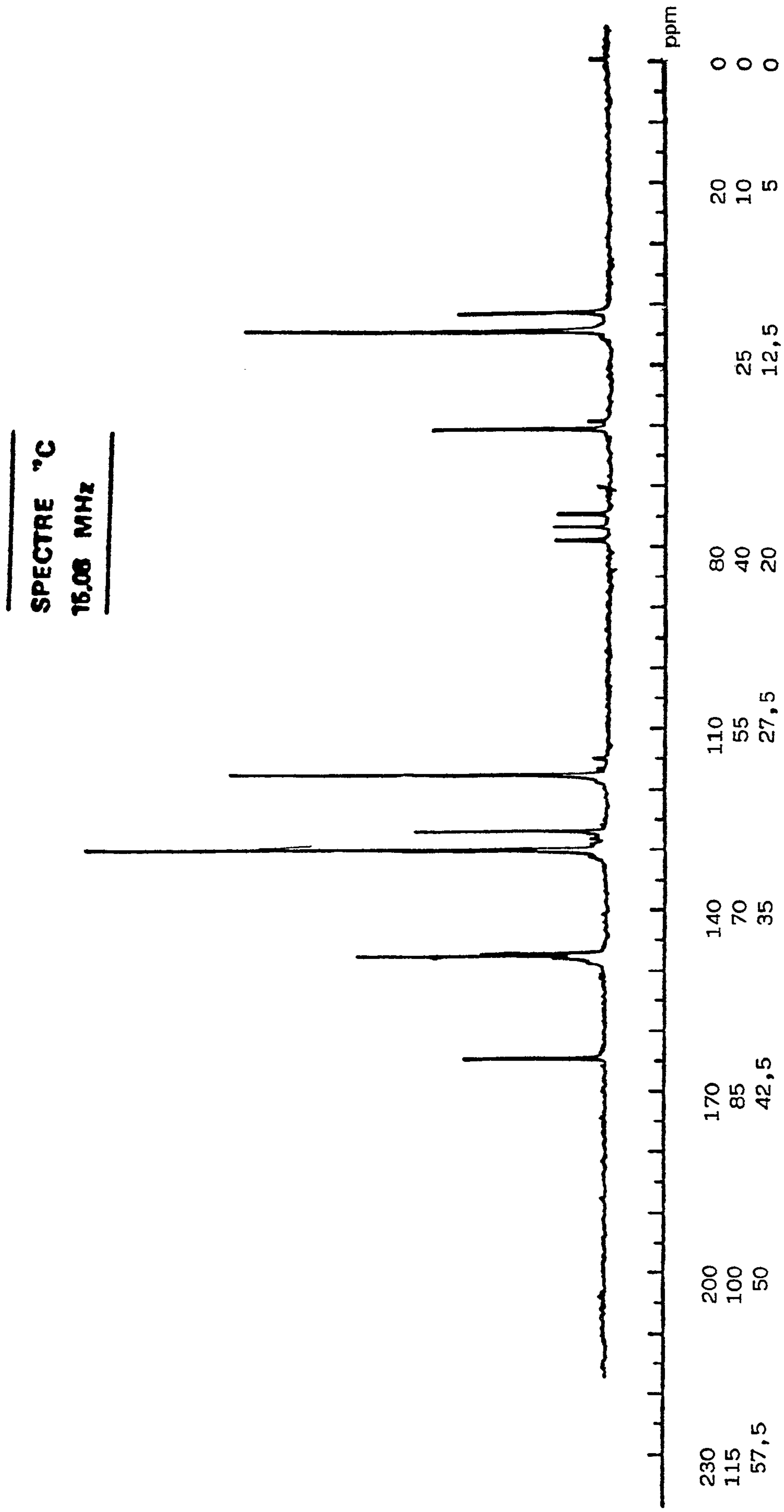
SPECTRE °C
15,08 MHz
ppm

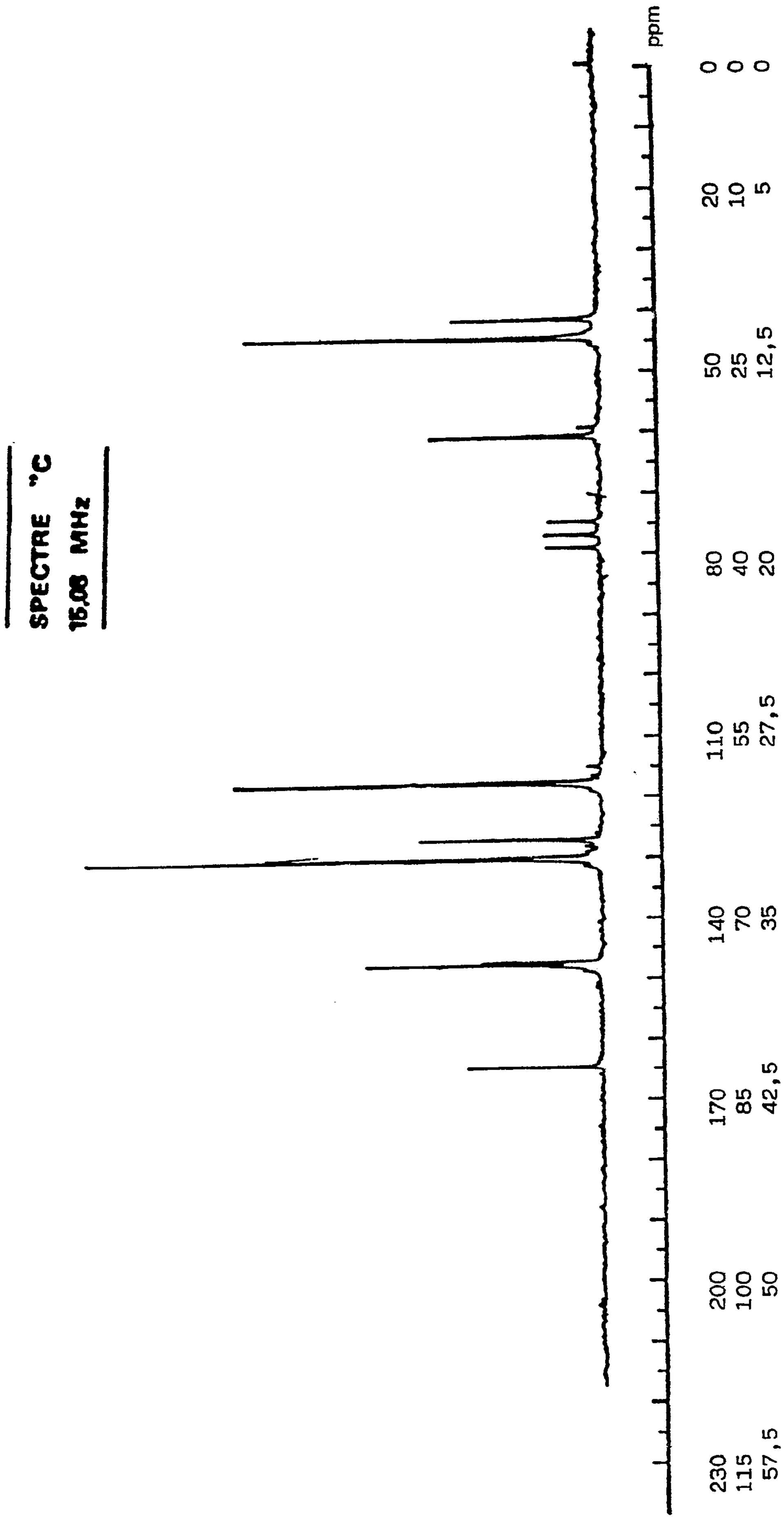
SPECTRE $^{13}$C
15,08 MHz
ppm
0 20 50 80 110 140 170 200 230
0 10 25 40 55 70 85 100 115
0 5 12,5 20 27,5 35 42,5 50 57,5